# NEW EUROPEAN PATENT SPECIFICATION

(11) **EP 2 175 908 B2**
(45) Date of publication and mention of the opposition decision: **21.08.2019**
(45) Mention of the grant of the patent: 17.08.2016
(21) Application number: 08762617.2
(22) Date of filing: 27.06.2008
(51) Int. Cl.: A61M 1/00, A61M 27/00

(54) **WOUND TREATMENT APPARATUS ABLE TO DISTINGUISH BETWEEN THE FAULT CONDITIONS "CANISTER FULL" AND "ASPIRANT CONDUIT BLOCKED"**
APPARAT ZUR WUNDBEHANDLUNG GEEIGNET DAFÜR ZWISCHEN DEN FEHLERZUSTÄNDEN "BEHÄLTER VOLL" UND "ABSAUGSCHLAUCH BLOCKIERT" ZU UNTERSCHEIDEN
APPAREIL POUR LE TRAITEMENT DES PLAIES APTE À DISTINGUER ENTRE LES ÉTATS D'ERREUR "RÉCIPIENT REMPLI" ET "CONDUIT D'ASPIRATION BLOQUÉ"

(30) Priority: 06.08.2007 GB 0715210
(43) Date of publication of application: 21.04.2010
(73) Proprietor: Smith & Nephew PLC, London WC2N 6LA (GB)
(72) Inventor: TURNER, Jake, Cambridge, Cambridgeshire CB23 3UG (GB); HARTWELL, Edward, Heslington, York YO10 5DF (GB); JACOB, Stephen, Fenstanton, Cambridge PE28 9JW (GB)
(74) Representative: HGF Limited
(86) International application number: PCT/GB2008/050515
(87) International publication number: WO 2009/019496

(56) References cited:
- WO-A-2006/105892
- WO-A-2008/036360
- WO-A-2008/048481
- WO-A2-2006/100053
- DE-A1-102005 014 420
- GB-A- 2 342 584
- US-A1- 2002 198 503
- US-A1- 2007 016 152
- US-A1- 2007 032 762
- US-A1- 2007 167 927
- US-A1- 2008 071 234
- US-A1- 2008 281 281

## Description

The present invention relates to a method for the application of topical negative pressure (TNP) therapy to wounds. In particular, but not exclusively, the present invention relates to the detection of blockages in TNP apparatus.

There is much prior art available relating to the provision of apparatus and methods of use thereof for the application of TNP therapy to wounds together with other therapeutic processes intended to enhance the effects of the TNP therapy. Examples of such prior art include those listed and briefly described below.

DE 10 2005 014420, GB2342584, WO2006/105892, US2008/071234, WO2008/048481, US2002/198503, US2007/016152 and WO2008/036360 describe various vacuum therapy devices.

TNP therapy assists in the closure and healing of wounds by reducing tissue oedema; encouraging blood flow and granulation of tissue; removing excess exudates and may reduce bacterial load and thus, infection to the wound. Furthermore, TNP therapy permits less outside disturbance of the wound and promotes more rapid healing.

In our co-pending International patent application, WO 2004/037334, apparatus, a wound dressing and a method for aspirating, irrigating and cleansing wounds are described. In very general terms, this invention describes the treatment of a wound by the application of topical negative pressure (TNP) therapy for aspirating the wound together with the further provision of additional fluid for irrigating and/or cleansing the wound, which fluid, comprising both wound exudates and irrigation fluid, is then drawn off by the aspiration means and circulated through means for separating the beneficial materials therein from deleterious materials. The materials which are beneficial to wound healing are recirculated through the wound dressing and those materials deleterious to wound healing are discarded to a waste collection bag or vessel.

In our co-pending International patent application, WO 2005/04670, apparatus, a wound dressing and a method for cleansing a wound using aspiration, irrigation and cleansing wounds are described. Again, in very general terms, the invention described in this document utilises similar apparatus to that in WO 2004/037334 with regard to the aspiration, irrigation and cleansing of the wound, however, it further includes the important additional step of providing heating means to control the temperature of that beneficial material being returned to the wound site/dressing so that it is at an optimum temperature, for example, to have the most efficacious therapeutic effect on the wound.

In our co-pending International patent application, WO 2005/105180, apparatus and a method for the aspiration, irrigation and/or cleansing of wounds are described. Again, in very general terms, this document describes similar apparatus to the two previously mentioned documents hereinabove but with the additional step of providing means for the supply and application of physiologically active agents to the wound site/dressing to promote wound healing.

The content of the above references is included herein by reference.

However, the above apparatus and methods are generally only applicable to a patient when hospitalised as the apparatus is complex, needing people having specialist knowledge in how to operate and maintain the apparatus, and also relatively heavy and bulky, not being adapted for easy mobility outside of a hospital environment by a patient, for example.

Some patients having relatively less severe wounds which do not require continuous hospitalisation, for example, but whom nevertheless would benefit from the prolonged application of TNP therapy, could be treated at home or at work subject to the availability of an easily portable and maintainable TNP therapy apparatus.

GB-A-2 307 180 describes a portable TNP therapy unit which may be carried by a patient clipped to belt or harness. In common with most prior art TNP therapy apparatus, the apparatus described cannot differentiate between a blocked or kinked aspiration conduit leading from a dressing to a waste canister and a blockage of the canister itself due to it being full, for example. The alarm to the user on this apparatus can be caused by a number of different faults or conditions.

It is an aim of the present invention to at least partly mitigate the above-mentioned problems.

It is an aim to provide apparatus which is able to distinguish between at least some distinct aspirant fluid non-flow conditions in TNP therapy apparatus so that a user is appropriately informed.

The disclosure encompasses a method of alerting a user of topical negative pressure therapy apparatus to a non-flow condition of aspirant fluid in the apparatus, the apparatus comprising a device having vacuum pump means and a waste canister connected to the device; the waste canister being operably connected to a wound dressing by aspiration conduit means for aspirating fluid from the wound; the aspiration conduit means, the waste canister and the device providing a fluid flow path therethrough and the vacuum pump means providing for fluid flow therethrough; the device having flowmeter means and first pressure sensing means at an upstream position between an outlet port for aspirated fluid from said waste canister and said pump means; said waste canister having further provision for selectable valve means for admission of ambient air to said waste canister; and, a control system for receiving signals from said flowmeter means and said pressure sensor and initiating control signals in response and activating an appropriate user alarm, said method comprising the steps of: measuring fluid flow and comparing actual fluid flow with a stored fluid flow value in control system memory means; opening said selectable valve means in response to actual fluid flow being below said stored value; measuring actual fluid flow after opening said selectable valve means; and activating an alarm corresponding to one of "canister full" and "aspirant conduit blocked" according to actual fluid flow value after opening of said selectable valve means.

According to the present invention there is provided a method of alerting a user of topical negative pressure therapy apparatus to a non-flow condition of aspirant fluid in the apparatus according to claim 1.

According to the present disclosure there is provided apparatus for alerting a user of topical negative pressure apparatus to a non-flow condition of aspirant fluid in the apparatus.

The invention is comprised in part of a method including apparatus for the provision of TNP therapy to a patient in almost any environment. The apparatus is lightweight, may be mains or battery powered by a rechargeable battery pack contained within a device (henceforth, the term "device" is used to connote a unit which may contain all of the control, power supply, power supply recharging, electronic indicator means and means for initiating and sustaining aspiration functions to a wound and any further necessary functions of a similar nature). When outside the home, for example, the apparatus may provide for an extended period of operation on battery power and in the home, for example, the device may be connected to the mains by a charger unit whilst still being used and operated by the patient.

The overall apparatus comprises: a dressing covering the wound and sealing at least an open end of an aspiration conduit to a cavity formed over the wound by the dressing; an aspiration tube comprising at least one lumen therethrough leading from the wound dressing to a waste material canister for collecting and holding wound exudates/waste material prior to disposal; and, a power, control and aspiration initiating and sustaining device associated with the waste canister.

The dressing covering the wound may be any type of dressing normally employed with TNP therapy and, in very general terms, may comprise, for example, a semi-permeable, flexible, self-adhesive drape material, as is known in the dressings art, to cover the wound and seal with surrounding sound tissue to create a sealed cavity or void over the wound. There may aptly be a porous barrier and support member in the cavity between the wound bed and the covering material to enable an even vacuum distribution to be achieved over the area of the wound. The porous barrier and support member being, for example, a gauze, a foam, an inflatable bag or known wound contact type material resistant to crushing under the levels of vacuum created and which permits transfer of wound exudates across the wound area to the aspiration conduit sealed to the flexible cover drape over the wound.

The aspiration conduit may be a plain flexible tube, for example, having a single lumen therethrough and made from a plastics material compatible with raw tissue, for example. However, the aspiration conduit may have a plurality of lumens therethrough to achieve specific objectives relating to the invention. A portion of the tube sited within the sealed cavity over the wound may have a structure to enable continued aspiration and evacuation of wound exudates without becoming constricted or blocked even at the higher levels of the negative pressure range envisaged.

It is envisaged that the negative pressure range for the apparatus embodying the present invention may be between about -50 mmHg and -200 mmHg (note that these pressures are relative to normal ambient atmospheric pressure thus, -200 mmHg would be about 560 mmHg in practical terms). Aptly, the pressure range may be between about -75 mmHg and -150 mmHg. Alternatively a pressure range of up to -75 mmHg, up to -80 mmHg or over -80 mmHg can be used. Also aptly a pressure range of below -75 mmHg could be used. Alternatively a pressure range of over -100 mmHg could be used or over -150 mmHg.

The aspiration conduit at its distal end remote from the dressing may be attached to the waste canister at an inlet port or connector. The device containing the means for initiating and sustaining aspiration of the wound/dressing may be situated between the dressing and waste canister, however, in the apparatus, the device may aspirate the wound/dressing via the canister thus, the waste canister may preferably be sited between the wound/dressing and device.

The aspiration conduit at the waste material canister end may preferably be bonded to the waste canister to prevent inadvertent detachment when being caught on an obstruction, for example.

The canister may be a plastics material moulding or a composite unit comprising a plurality of separate mouldings. The canister may aptly be translucent or transparent in order to visually determine the extent of filling with exudates. However, the canister and device may in some embodiments provide automatic warning of imminent canister full condition and may also provide means for cessation of aspiration when the canister reaches the full condition.

The canister may be provided with filters to prevent the exhaust of liquids and odours therefrom and also to prevent the expulsion of bacteria into the atmosphere. Such filters may comprise a plurality of filters in series. Examples of suitable filters may comprise hydrophobic filters of 0.2µm pore size, for example, in respect of sealing the canister against bacteria expulsion and 1 µm against liquid expulsion.

Aptly, the filters may be sited at an upper portion of the waste canister in normal use, that is when the apparatus is being used or carried by a patient the filters are in an upper position and separated from the exudate liquid in the waste canister by gravity. Furthermore, such an orientation keeps the waste canister outlet or exhaust exit port remote from the exudate surface.

Aptly the waste canister may be filled with an absorbent gel such as ISOLYSEL (trade mark), for example, as an added safeguard against leakage of the canister when full and being changed and disposed of. Added advantages of a gel matrix within the exudate storing volume of the waste canister are that it prevents excessive movement, such as slopping, of the liquid, minimises bacterial growth and minimises odours.

The waste canister may also be provided with suitable means to prevent leakage thereof both when detached from the device unit and also when the aspiration conduit is detached from the wound site/dressing.

The canister may have suitable means to prevent emptying by a user (without tools or damage to the canister) such that a full or otherwise end-of-life canister may only be disposed of with waste fluid still contained.

The device and waste canister may have mutually complementary means for connecting a device unit to a waste canister whereby the aspiration means in the device unit automatically connects to an evacuation port on the waste canister such that there is a continuous aspiration path from the wound site/dressing to an exhaust port on the device.

Aptly, the exhaust port from the fluid path through the apparatus is provided with filter means to prevent offensive odours from being ejected into the atmosphere.

In general terms the device unit comprises an aspirant pump; means for monitoring pressure applied by the aspirant pump; may have a flowmeter to monitor fluid flow through the aspirant pump; a control system which controls the aspirant pump in response to signals from sensors such as the pressure monitoring means and the flowmeter, for example, and which control system also controls a power management system with regard to an on-board battery pack and the charging thereof and lastly a user interface system whereby various functions of the device such as pressure level set point, for example, may be adjusted (including stopping and starting of the apparatus) by a user. The device unit may contain all of the above features within a single unified casing.

In view of the fact that the device unit contains the majority of the intrinsic equipment cost therein ideally it will also be able to survive impact, tolerate cleaning in order to be reusable by other patients.

In terms of pressure capability the aspiration means may be able to apply a maximum pressure drop of at least -200 mmHg to a wound site/dressing. The apparatus is capable of maintaining a predetermined negative pressure even under conditions where there is a small leak of air into the system and a high exudate flow.

The pressure control system may prevent the minimum pressure achieved from exceeding for example -200 mmHg so as not to cause undue patient discomfort. The pressure required may be set by the user at a number of discreet levels such as -50, -75, -100, -125, -150, -175 mmHg, for example, depending upon the needs of the wound in question and the advice of a clinician. Thus suitable pressure ranges in use may be from -25 to -80 mmHg, or -50 to -76 mmHg, or -50 to -75 mmHg as examples. The control system may also advantageously be able to maintain the set pressure within a tolerance band of +/- 10 mmHg of the set point for 95% of the time the apparatus is operating given that leakage and exudation rates are within expected or normal levels.

Aptly, the control system may trigger alarm means such as a flashing light, buzzer or any other suitable means when various abnormal conditions apply such as, for example: pressure outside set value by a large amount due to a gross leak of air into system; duty on the aspiration pump too high due to a relatively smaller leakage of air into the system; pressure differential between wound site and pump is too high due, for example, to a blockage or waste canister full.

In prior art TNP apparatus blockages in the waste canister filter due to full canister, for example, or a blocked aspirant conduit due to waste exudate or a kink in the conduit can be detected by measuring differential pressure readings or by using a flow meter. However, this is not sufficient to be able to distinguish whether the cause of a decrease in aspirant fluid flow rate is a full waste canister or a blocked aspirant conduit. In the apparatus of the present disclosure it is desired to be able to distinguish between various aspirant fluid non-flow conditions and to be able to alert a user with a specific warning appropriate to the specific condition or fault.

In one aspect, the waste canister is provided with a further port having an air access tube and/or a selectable valve associated therewith. The selectable valve may be a solenoid type valve, for example, and be selected in accordance with control responses from the control system. The port may be opened to atmosphere by opening of the selectable valve. When the control system senses a blockage by, for example, a fall in aspirated fluid flow rate through the fluid flow path, the control system opens the valve so as to admit air into the waste canister (it will be understood that the interior of the waste canister forms part of the fluid flow path through the apparatus, the fluid flow path extending from the end of the aspirant conduit at the wound site/dressing to the final exhaust port venting aspirated gaseous fluid to atmosphere in the device). If the canister is full then the effect that opening this port and the subsequent air flow into the canister will have on the flow meter located in the device and which is in electrical connection with the control system, will be much less than the effect which would pertain if the fault is a blocked aspirant conduit. If the aspirant conduit between the waste canister and the wound site/dressing is blocked by waste matter or the conduit is kinked, for example, the blockage measured by the flowmeter will effectively disappear as inflowing air into the canister via the selectable valve will restore the fluid flow rate in the fluid flow path. If, however, the blockage is due to a full waste canister or a blocked waste canister filter adjacent the fluid outlet port in the waste canister then opening the valve will have little or no effect on the fluid flow rate through the fluid flow path. Thus depending upon the resulting effect of opening the selectable valve to atmosphere, the control system can determine the cause and location of the blockage and alert the user appropriately. This is important to a user since if the problem is merely a kinked or twisted or otherwise caught aspirant conduit then the user is able to instantly remedy the fault and has no need to check if the canister is full.

Where a selectable valve is employed in the waste canister to admit ambient air therein it is preferred that the opening so formed is provided with appropriate filters so as to prevent any possible ejection of waste material and/or bacteria from the canister.

In the control system memory a pressure value for a blocked system (from any cause) is defined. At periodic intervals, whilst the apparatus is in use, the control system samples the pressure at the sensor upstream of the pump and compares that pressure with the stored blocked system value and takes appropriate action when certain criteria are fulfilled.

The two pressure sensors need to detect whether the canister is full or if the aspirant conduit is blocked. Under normal operating conditions the first pressure at the pump inlet will be whatever the set pressure may be and the second pressure in the canister will be somewhat lower, for example about 20mmHg less (i.e. 20mmHg less negative), due to the pressure drop caused by the flow of fluid through the filter at the canister exit port. Thus, when the apparatus is operating normally with no blockages of any kind the pressure difference between first and second pressures may be of the order of +20mmHg. When the canister is full and can no longer transmit aspirant (gaseous) fluid through the filter to the canister exit port due to the filter being occluded by waste material, the pressure in the canister will eventually rise towards atmospheric as the pressure in the aspirant conduit connected to the dressing will rise due to no vacuum being applied by the pump due to the occluded canister filter.

Consequently, the pressure difference will rise (increase in the negative direction if being measured relative to atmosphere) to a difference significantly more than that pertaining under normal operating conditions thus, the control system will recognise diverging first and second pressures as indicating a full canister. However, in the case where the aspirant conduit is blocked by kinking or twisting, for example, the pressure difference will diminish between first and second pressures as the vacuum level in the canister (second pressure) will decrease to approach that at the pump inlet (first pressure). Consequently, the control system will recognise converging first and second pressures as indicating a blocked aspirant conduit. Depending upon which condition exists an appropriate alarm will be activated.

The "stored value" in the control system may be a range or tolerance band of pressures rather than a single unique pressure to permit more meaningful operation of the two conditions described above. Thus, under "normal" operating conditions the control system will recognise pressures lying within a stored range.

A flowmeter means may be employed, the control system memory also having a stored value of fluid flow below which a potential blockage is recognised.

A further pressure sensor may be provided to sense pressure at the wound site/dressing. The apparatus of the present invention may be provided with a carry case and suitable support means such as a shoulder strap or harness, for example. The carry case may be adapted to conform to the shape of the apparatus comprised in the joined together device and waste canister. In particular, the carry case may be provided with a bottom opening flap to permit the waste canister to be changed without complete removal of the apparatus form the carry case.

The carry case may be provided with an aperture covered by a displaceable flap to enable user access to a keypad for varying the therapy applied by the apparatus.

In order that the present invention may be more fully understood, examples will now be described by way of illustration only with reference to the accompanying drawings, of which:
Figure 1 shows a generalised schematic block diagram showing a general view of an apparatus and the constituent apparatus features thereof;
Figure 2 shows a similar generalised schematic block diagram to Figure 1 and showing fluid paths therein;
Figure 3 shows a generalised schematic block diagram similar to Figure 1 but of a device unit only and showing power paths for the various power consuming/producing features of the apparatus;
Figure 4 shows a similar generalised schematic block diagram to Figure 3 of the device unit and showing control system data paths for controlling the various functions and components of the apparatus;
Figure 5 shows a perspective view of an apparatus;
Figure 6 shows a perspective view of an assembled device unit of the apparatus of Figure 5;
Figure 7 shows an exploded view of the device unit of Figure 6;
Figure 8 shows a partially sectioned side elevation view through the interface between a waste canister and device unit of the apparatus;
Figure 9 shows a cross section through a waste canister of the apparatus of Figures 5 to 8;
Figure 10 shows a schematic diagram of apparatus;
Figure 11 shows a schematic diagram of apparatus according to the present invention;
Figures 12A and 12B show graphs of sample pressure readings from the two pressure sensors in the second embodiment shown in Figure 11 and determine the cause of a blockage; and
Figure 13 which shows a modified embodiment of that shown in Figure 10.

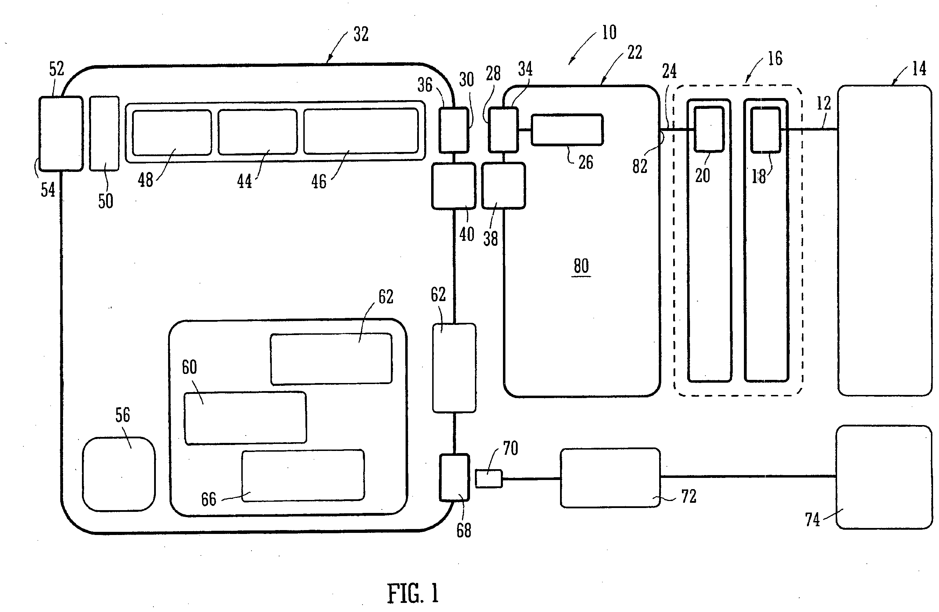

Referring now to Figures 1 to 4 of the drawings and where the same or similar features are denoted by common reference numerals.

Figure 1 shows a generalised schematic view of an apparatus 10 of a portable topical negative pressure (TNP) system. It will be understood that embodiments of the present invention are generally applicable to use in such a TNP system. Briefly, negative pressure wound therapy assists in the closure and healing of many forms of "hard to heal" wounds by reducing tissue oedema; encouraging blood flow and granular tissue formation; removing excess exudate and may reduce bacterial load (and, therefore, infection). In addition the therapy allows for less disturbance of a wound leading to more rapid healing. The TNP system is detailed further hereinafter but in summary includes a portable body including a canister and a device with the device capable of providing an extended period of continuous therapy within at least a one year life span. The system is connected to a patient via a length of tubing with an end of the tubing operably secured to a wound dressing on the patient.

More particularly, as shown in Figure 1, the apparatus comprises an aspiration conduit 12 operably and an outer surface thereof at one end sealingly attached to a dressing 14. The dressing 14 will not be further described here other than to say that it is formed in a known manner from well know materials to those skilled in the dressings art to create a sealed cavity over and around a wound to be treated by TNP therapy with the apparatus of the present invention. The aspiration conduit has an in-line connector 16 comprising connector portions 18, 20 intermediate its length between the dressing 14 and a waste canister 22. The aspiration conduit between the connector portion 20 and the canister 22 is denoted by a different reference numeral 24 although the fluid path through conduit portions 12 and 24 to the waste canister is continuous. The connector portions 18, 20 join conduit portions 12, 24 in a leak-free but disconnectable manner. The waste canister 22 is provided with filters 26 which prevent the escape via an exit port 28 of liquid and bacteria from the waste canister. The filters may comprise a 1µm hydrophobic liquid filter and a 0.2µm bacteria filter such that all liquid and bacteria is confined to an interior waste collecting volume of the waste canister 22. The exit port 28 of the waste canister 22 mates with an entry/suction port 30 of a device unit 32 by means of mutually sealing connector portions 34, 36 which engage and seal together automatically when the waste canister 22 is attached to the device unit 32, the waste canister 22 and device unit 32 being held together by catch assemblies 38, 40. The device unit 32 comprises an aspirant pump 44, an aspirant pressure monitor 46 and an aspirant flowmeter 48 operably connected together. The aspiration path takes the aspirated fluid which in the case of fluid on the exit side of exit port 28 is gaseous through a silencer system 50 and a final filter 52 having an activated charcoal matrix which ensures that no odours escape with the gas exhausted from the device 32 via an exhaust port 54. The filter 52 material also serves as noise reducing material to enhance the effect of the silencer system 50. The device 32 also contains a battery pack 56 to power the apparatus which battery pack also powers the control system 60 which controls a user interface system 62 controlled via a keypad (not shown) and the aspiration pump 44 via signals from sensors 46, 48. A power management system 66 is also provided which controls power from the battery pack 56, the recharging thereof and the power requirements of the aspirant pump 44 and other electrically operated components. An electrical connector 68 is provided to receive a power input jack 70 from a SELV power supply 72 connected to a mains supply 74 when the user of the apparatus or the apparatus itself is adjacent a convenient mains power socket.

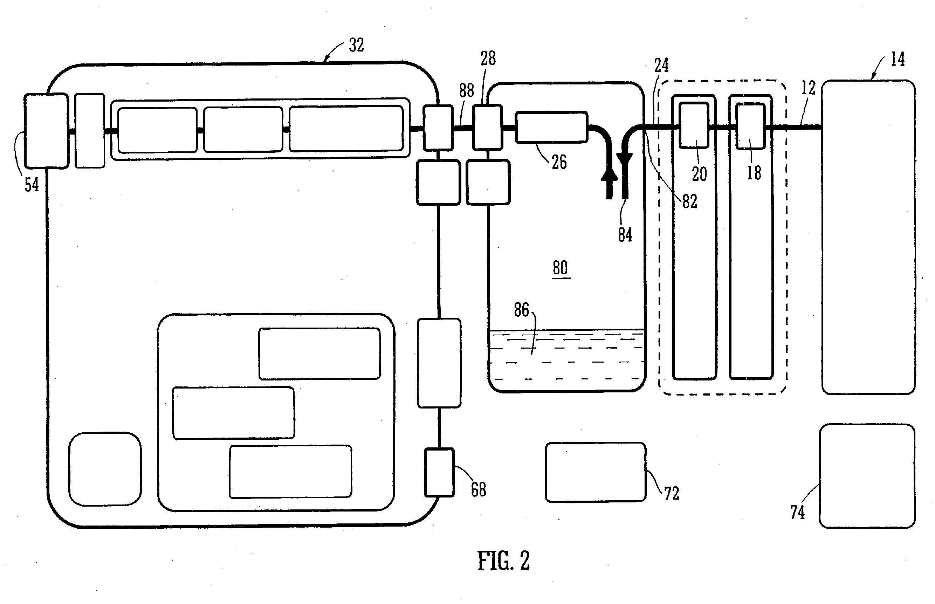

Figure 2 shows a similar schematic representation to Figure 1 but shows the fluid paths in more detail. The wound exudate is aspirated from the wound site/dressing 14 via the conduit 12, the two connector portions 18, 20 and the conduit 24 into the waste canister 22. The waste canister 22 comprises a relatively large volume 80 in the region of 500ml into which exudate from the wound is drawn by the aspiration system at an entry port 82. The fluid 84 drawn into the canister volume 80 is a mixture of both air drawn into the dressing 14 via the semi-permeable adhesive sealing drape (not shown) and liquid 86 in the form of wound exudates. The volume 80 within the canister is also at a lowered pressure and the gaseous element 88 of the aspirated fluids is exhausted from the canister volume 80 via the filters 26 and the waste canister exhaust exit port 28 as bacteria-free gas. From the exit port 28 of the waste canister to the final exhaust port 54 the fluid is gaseous only.

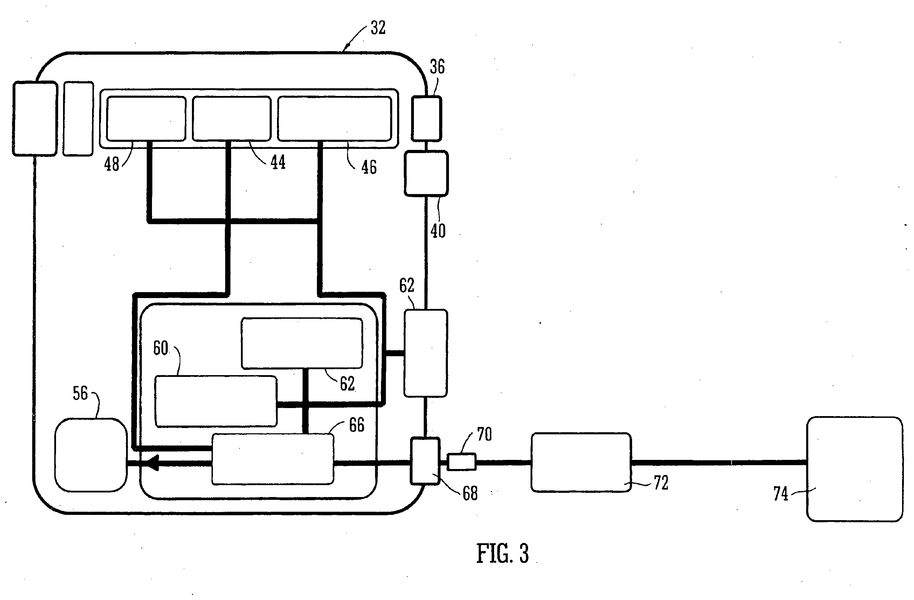

Figure 3 shows a schematic diagram showing only the device portion of the apparatus and the power paths in the device of the apparatus embodying the present invention.

Power is provided mainly by the battery pack 56 when the user is outside their home or workplace, for example, however, power may also be provided by an external mains 74 supplied charging unit 72 which when connected to the device 32 by the socket 68 is capable of both operating the device and recharging the battery pack 56 simultaneously. The power management system 66 is included so as to be able to control power of the TNP system. The TNP system is a rechargeable, battery powered system but is capable of being run directly from mains electricity as will be described hereinafter more fully with respect to the further figures. If disconnected from the mains the battery has enough stored charge for approximately 8 hours of use in normal conditions. It will be appreciated that batteries having other associated life times between recharge can be utilised. For example batteries providing less than 8 hours or greater than 8 hours can be used. When connected to the mains the device will run off the mains power and will simultaneously recharge the battery if depleted from portable use. The exact rate of battery recharge will depend on the load on the TNP system. For example, if the wound is very large or there is a significant leak, battery recharge will take longer than if the wound is small and well sealed.

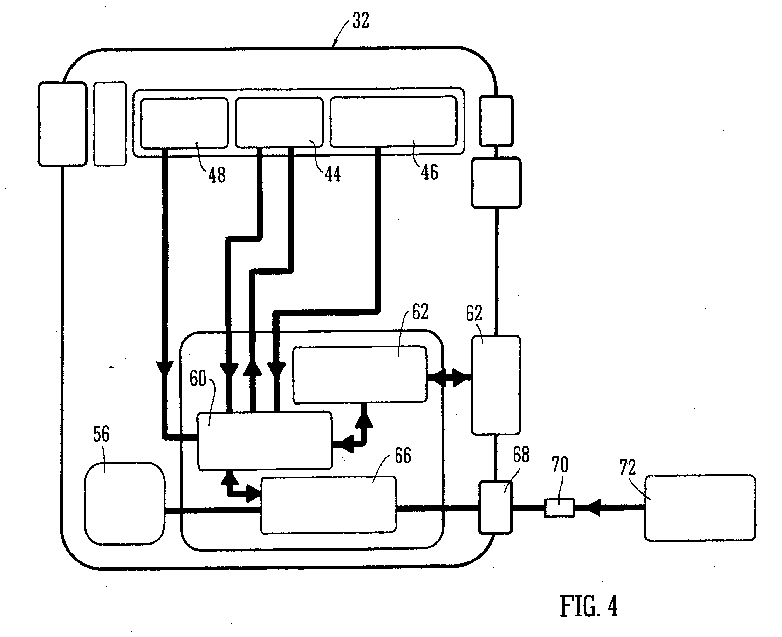

Figure 4 shows the device 32 part of the apparatus embodying the present invention and the data paths employed in the control system for control of the aspirant pump and other features of the apparatus. A key purpose of the TNP system is to apply negative pressure wound therapy. This is accomplished via the pressure control system which includes the pump and a pump control system. The pump applies negative pressure; the pressure control system gives feedback on the pressure at the pump head to the control system; the pump control varies the pump speed based on the difference between the target pressure and the actual pressure at the pump head. In order to improve accuracy of pump speed and hence provide smoother and more accurate application of the negative pressure at a wound site, the pump is controlled by an auxiliary control system. The pump is from time to time allowed to "free-wheel" during its duty cycle by turning off the voltage applied to it. The spinning motor causes a "back electro-motive force" or BEMF to be generated. This BEMF can be monitored and can be used to provide an accurate measure of pump speed. The speed can thus be adjusted more accurately than can prior art pump systems.

According to embodiments of the present invention, actual pressure at a wound site is not measured but the difference between a measured pressure (at the pump) and the wound pressure is minimised by the use of large filters and large bore tubes wherever practical. If the pressure control measures that the pressure at the pump head is greater than a target pressure (closer to atmospheric pressure) for a period of time, the device sends an alarm and displays a message alerting the user to a potential problem such as a leak.

In addition to pressure control a separate flow control system can be provided. A flow meter may be positioned after the pump and is used to detect when a canister is full or the tube has become blocked. If the flow falls below a certain threshold, the device sounds an alarm and displays a message alerting a user to the potential blockage or full canister.

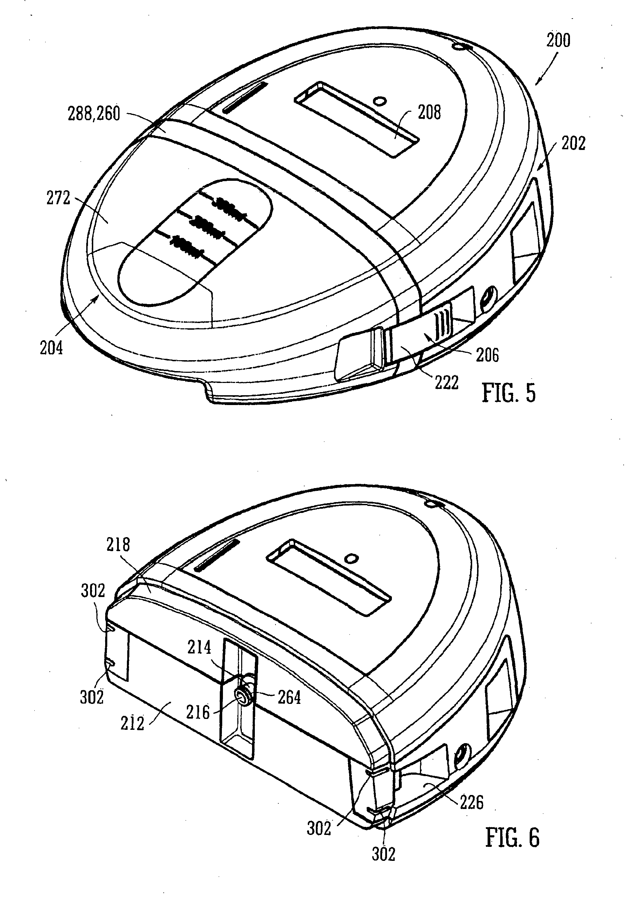

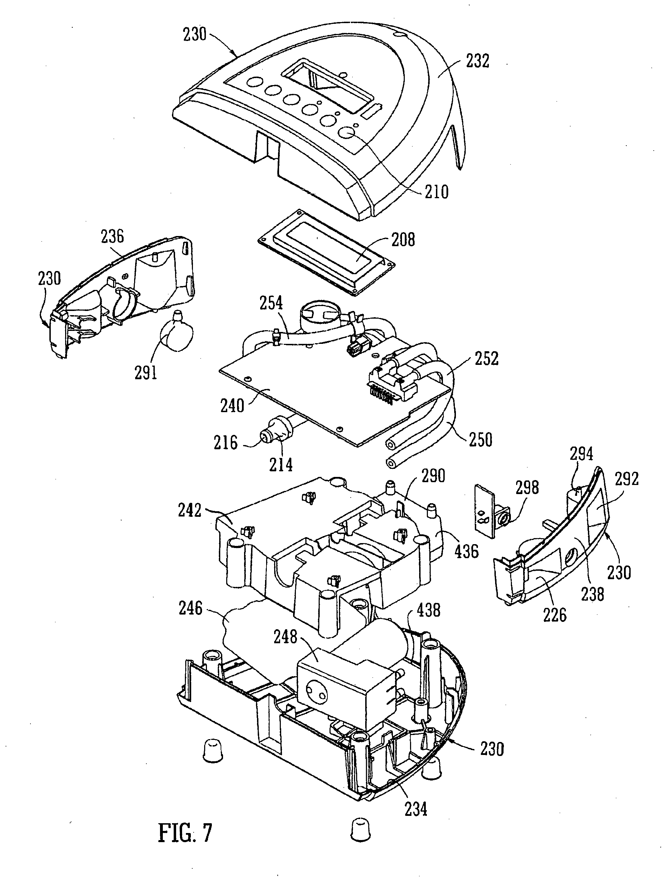

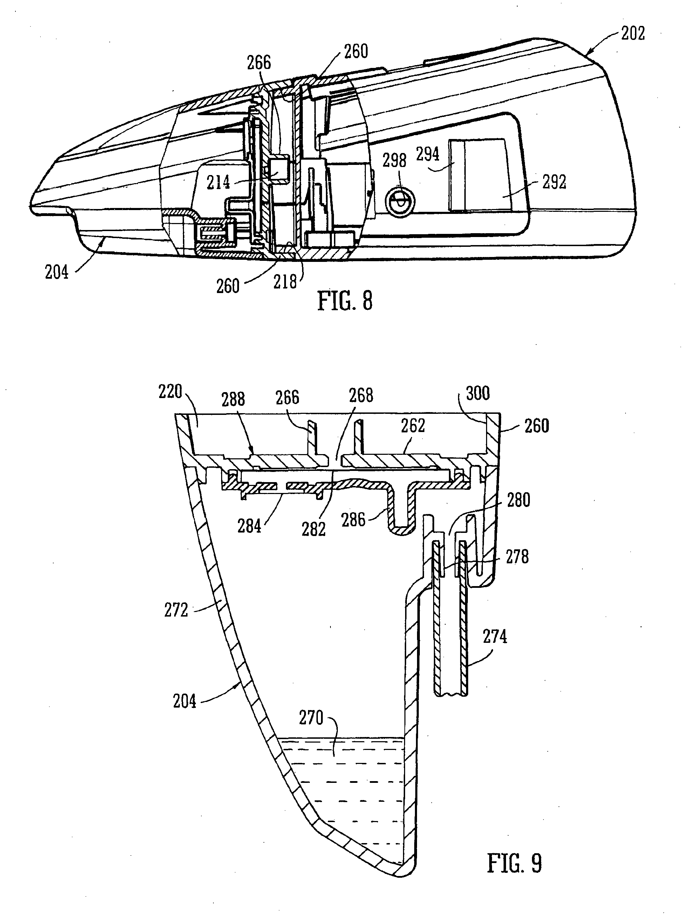

Referring now to Figures 5 to 9 which show various views and cross sections of a preferred embodiment of apparatus 200 embodying the present invention. The preferred embodiment is of generally oval shape in plan and comprises a device unit 202 and a waste canister 204 connected together by catch arrangements 206. The device unit 202 has a liquid crystal display (LCD) 208, which gives text based feedback on the wound therapy being applied, and a membrane keypad 210, the LCD being visible through the membrane of the keypad to enable a user to adjust or set the therapy to be applied to the wound (not shown). The device has a lower, generally transverse face 212 in the centre of which is a spigot 214 which forms the suction/entry port 216 to which the aspiration means (to be described below) are connected within the device unit. The lower edge of the device unit is provided with a rebated peripheral male mating face 218 which engages with a co-operating peripheral female formation 220 on an upper edge of the waste canister 204 (see Figures 8 and 9). On each side of the device 202, clips 222 hinged to the canister 204 have an engaging finger (not shown) which co-operates with formations in recesses 226 in the body of the device unit. From Figure 7 it may be seen that the casing 230 of the device unit is of largely "clamshell" construction comprising front and back mouldings 232, 234, respectively and left-hand and right-hand side inserts 236, 238. Inside the casing 230 is a central chassis 240 which is fastened to an internal moulded structural member 242 and which chassis acts as a mounting for the electrical circuitry and components and also retains the battery pack 246 and aspiration pump unit 248. Various tubing items 250, 252, 254 connect the pump unit 248 and suction/entry port 216 to a final gaseous exhaust via a filter 290. Figure 8 shows a partially sectioned side elevation of the apparatus 200, the partial section being around the junction between the device unit 202 and the waste canister 204, a cross section of which is shown at Figure 9. Theses views show the rebated edge 218 of the male formation on the device unit co-operating with the female portion 220 defined by an upstanding flange 260 around the top face 262 of the waste canister 204. When the waste canister is joined to the device unit, the spigot 214 which has an "O" ring seal 264 therearound sealingly engages with a cylindrical tube portion 266 formed around an exhaust/exit port 268 in the waste canister. The spigot 214 of the device is not rigidly fixed to the device casing but is allowed to "float" or move in its location features in the casing to permit the spigot 214 and seal 264 to move to form the best seal with the bore of the cylindrical tube portion 266 on connection of the waste canister to the device unit. The waste canister 204 in Figure 9 is shown in an upright orientation much as it would be when worn by a user. Thus, any exudate 270 would be in the bottom of the internal volume of waste receptacle portion 272. An aspiration conduit 274 is permanently affixed to an entry port spigot 278 defining an entry port 280 to receive fluid aspirated from a wound (not shown) via the conduit 274. Filter members 282 comprising a 0.2µm filter and 284 comprising a 1µm filter are located by a filter retainer moulding 286 adjacent a top closure member or bulkhead 288 the filter members preventing any liquid or bacteria from being drawn out of the exhaust exit port 268 into the pump and aspiration path through to an exhaust and filter unit 290 which is connected to a casing outlet moulding at 291 via an exhaust tube (not shown) in casing side piece 236. The side pieces 236, 238 are provided with recesses 292 having support pins 294 therein to locate a carrying strap (not shown) for use by the patient. The side pieces 230 and canister 204 are also provided with features which prevent the canister and device from exhibiting a mutual "wobble" when connected together. Ribs (not shown) extending between the canister top closure member 288 and the inner face 300 of the upstanding flange 260 locate in grooves 302 in the device sidewalls when canister and device are connected. The casing 230 also houses all of the electrical equipment and control and power management features, the functioning of which was described briefly with respect to Figures 3 and 4 hereinabove. The side piece 238 is provided with a socket member 298 to receive a charging jack from an external mains powered battery charger (both not shown).

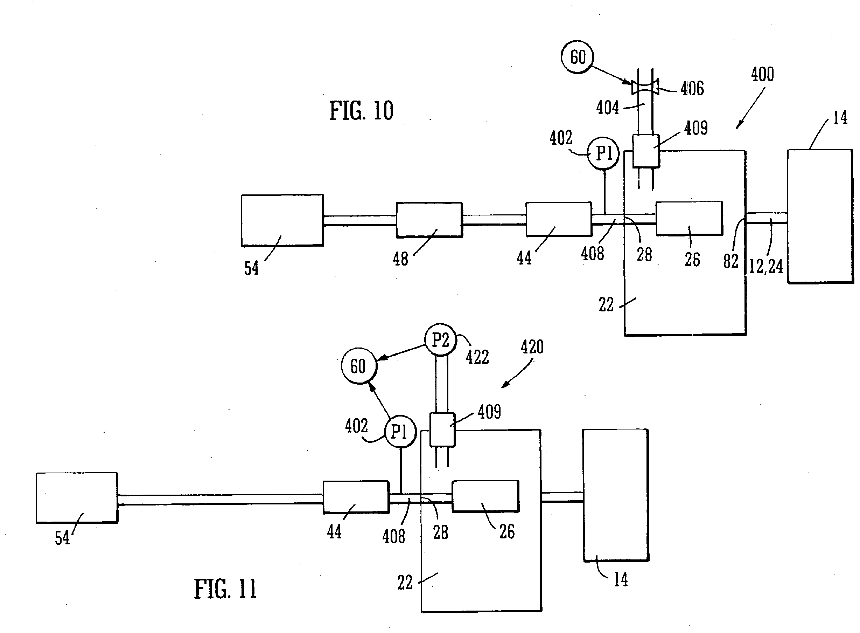

Referring now to Figures 10 to 12 and where the same or similar features are denoted by common reference numerals. Reference to Figures 1 to 4 may also be made in the following description.

Figure 10 shows a schematic diagram of apparatus 400. The apparatus includes an aspiration conduit 12, 24 connected to a dressing 14; the aspiration conduit 24 being connected to a waste canister 22 having a filter 26 adjacent an outlet port 28; a device which houses an aspirant pump 44 and a control system (not shown in Fig. 10 but see 60 in Figs 1 to 4). The device has a pressure sensor 402 at a position upstream of the pump 44 and in between the pump 44 and the outlet port 28 of the waste canister 22, the waste canister and pump being connected by tubing 408 forming part of the aspirant fluid flow path. A flowmeter 48 is provided downstream of the pump 44 (but may, however, be sited upstream of the pump between the waste canister and pump). Downstream of the waste canister outlet port 28 the fluid flow is gaseous as all liquid and bacteria is retained in the canister by the filters 26 (see also 282 in Fig.9), the pressure sensor 402 being sited in this gaseous fluid flow region. The canister is also provided with a relief conduit 404 having a selectable valve 406, such as a solenoid valve, for example, therein which is able to open the relief line 404 to atmosphere (note that access of air to the waste canister through the line 404 and valve 406 is directly thereto and not via the filters 26 in the waste canister), the relief line 404 having a separate filter 409 therein to prevent ejection of any waste matter or bacteria to the atmosphere when the valve 406 is open. The selectable valve 406 is electrically connected to the control system 60 which is able to open and close the valve in response to pressure signals from the sensor 402. In response to the device control system 60 sensing a full canister or filter blockage or suchlike, due to the fluid flow rate sensed by the flowmeter 48 decreasing blow a stored value in the control system memory, the control system 60 opens the valve 406 to allow air to flow into the interior of the waste canister since the waste canister interior is connected to the vacuum pump as it is part of the fluid flow path and the pressure therein is negative with respect to ambient air pressure. If the canister 22 is full the effect that the airflow into the canister will have on the flowmeter 48 (see Figs 1 to 4) in the fluid flow path will be relatively small and the control system will interpret this as a canister full condition and activate an appropriate alarm to the user. If, however, the opening of the valve 406 results in normal fluid flow rate resuming then this is interpreted by the control system as a blocked or otherwise closed-off aspirant conduit since the inflow of air via the valve 406 will immediately restore normal fluid flow rate as sensed by the flowmeter 48. In this latter case the control system will activate an alarm appropriate to a blocked conduit condition.

Sample psuedo code for a system incorporating the relief conduit 404 and valve 406 with pressure and flow measurement for blockage detection is given below:

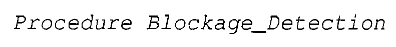

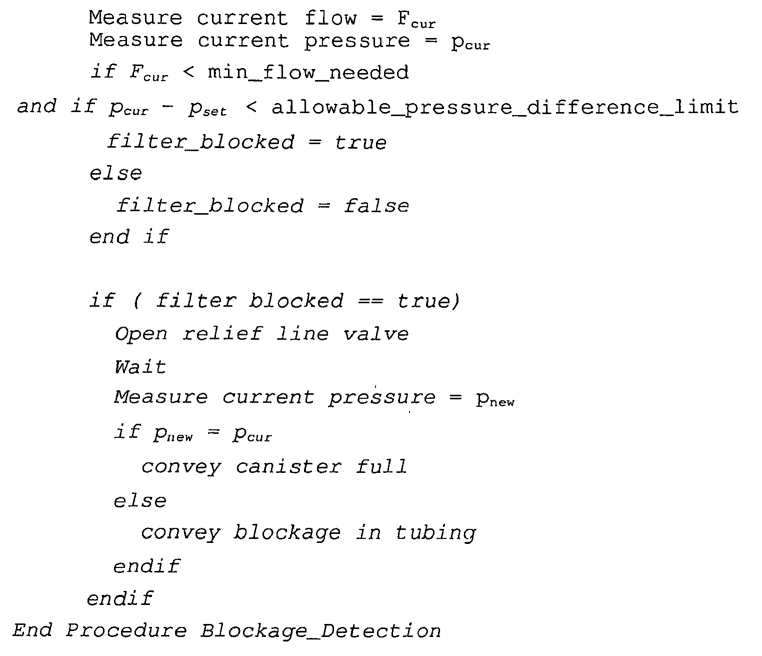

As will be known to those skilled in the control system art, the steps recited above may be repeated by the control system software at predetermined intervals all the time the TNP apparatus is in use.

Figure 11 shows a schematic diagram of apparatus and corresponds to the method of the present invention. Again, reference is also made to Figures 1 to 4. In this embodiment 420 a second pressure sensor 422 is situated so as to be able to monitor the pressure inside the waste canister upstream of the filters 26. The control system 60 monitors and compares the pressure readings from sensors 402 and 422, the level of pressure differential indicating the type of blockage which exists. If the pressure differential is low relative to a stored value or value range in the control system memory then the control system interprets this as a blocked aspirant conduit since the vacuum level in the canister is comparable to that at the pump inlet. If the pressure differential is larger than the stored value or value range in the control system memory, then the control system interprets this as a blocked filter in the canister due to the canister being full and unable to maintain the vacuum. The control system activates the appropriate alarm to the user whichever condition pertains. In this embodiment the flowmeter 48 of the first embodiment may not be needed and may be dispensed with to reduce cost.

Sample psuedo code for a system incorporating the differential pressure measurement method for blockage detection is given below:

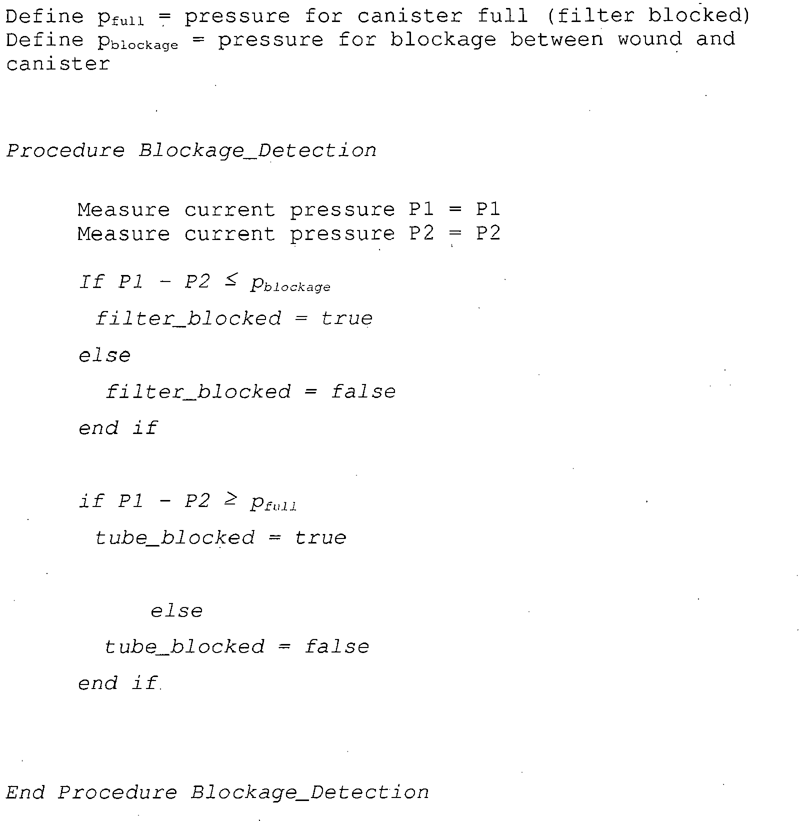

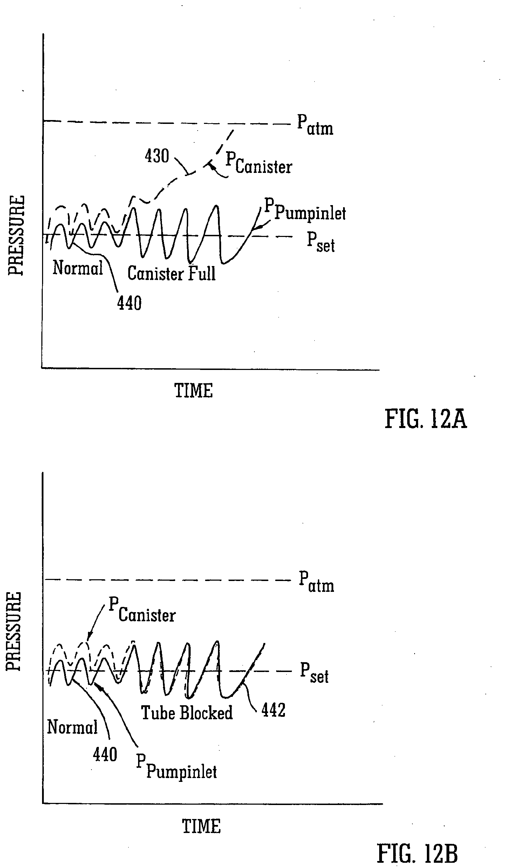

Figures 12A and 12B show graphs giving sample readings from the two sensors 402, 422. The solid line labelled "Ppumpinlet" indicates the pressure readings from sensor 402 and the broken line labelled "Pcanister" indicates the pressure readings from sensor 422. The graph indicates "Pset" which is the value or tolerance range stored in the control system memory and "Patm" which is ambient atmospheric pressure outside the canister. Under normal operating conditions, i.e. when there is no blockage of any kind, the canister being less than full and the aspirant conduit 12, 24 being open to unimpeded flow other than to usual viscosity effects and conduit interaction with the flowing fluid, the pressure monitored at the first pressure sensor 402 is at or about a preset value 440 to apply vacuum to the wound site/dressing 14 and, the pressure sensed by the second sensor 422 is at a lower negative pressure due to the pressure drop caused by the filter 26. The pressure differential between sensors 402 and 422 may be about +20mmHg, for example, when the apparatus is operating normally. When pressure Pcanister rises above a pre-stored tolerance level as at 430 then the control system interprets this as a canister full condition. In contrast to this, and as shown in the graph of Fig. 12B, when the aspirant conduit is blocked the normal pressure difference of about +20mmHg falls further and the first and second pressures converge towards each other as at 442 and the control system recognises this as a blocked aspirant conduit condition and activates an appropriate alarm.

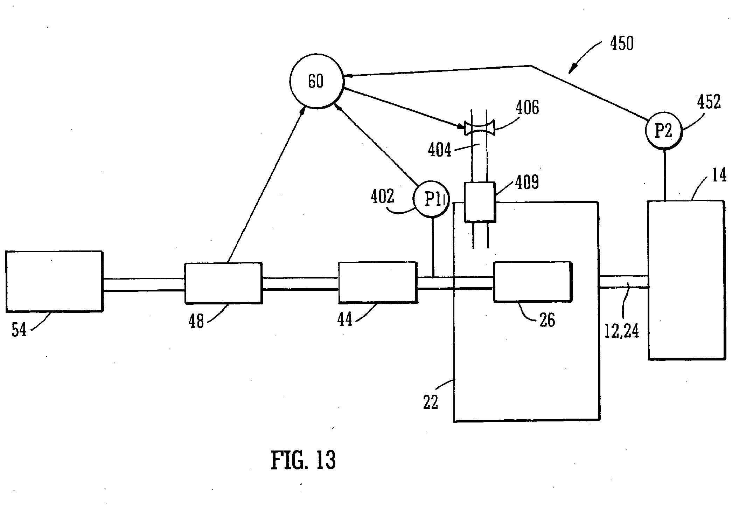

Figure 13 shows a modified embodiment 450 similar to that shown and described with reference to Fig. 10. However, in this modified embodiment a second pressure sensor 452 is provided, both the first second pressure sensors 402 and 452 and the flowmeter 48 being in electrical connection to the control system 60 for transmitting signals thereto. The control system 60 is also electrically connected to the valve 406 for opening and closing thereof. In this embodiment the control system 60 monitors the pressures from the sensors 402 and 452 and when the pressure differential exceeds a stored value in the control system memory by a greater negative amount the control system opens the valve 406 to admit ambient air to the waste canister 22. Depending upon the effect of the admitted air on the fluid flow rate sensed by the flowmeter 48 the control system determines the cause of the blockage as in the description with reference to Figure 10.

Throughout the description and claims of this specification, the words "comprise" and "contain" and variations of the words, for example "comprising" and "comprises", means "including but not limited to", and is not intended to (and does not) exclude other moieties, additives, components, integers or steps.

Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith.

## Claims

1. A method of alerting a user of topical negative pressure therapy apparatus to a non-flow condition of aspirant fluid in the apparatus, the apparatus comprising a device (32) having vacuum pump means (44) and a waste canister (22) connected to the device; the waste canister being operably connected to a wound dressing (14) by aspiration conduit means (12, 24) for aspirating fluid from the wound; the aspiration conduit means, the waste canister and the device providing a fluid flow path therethrough and the vacuum pump means providing for fluid flow therethrough; the device having first pressure sensing means (402) between an outlet port for aspirated fluid from said waste canister and said pump means; said waste canister having second pressure sensor means (422, 452) associated therewith; and, a control system (60) for receiving signals from said first and second pressure sensor means and initiating control signals in response to said signals and activating an appropriate user alarm, measuring pressure at said first pressure sensor and at said second pressure sensor; comparing said first and said second pressures; if a pressure difference between first and second pressures is greater than a stored value in a memory of said control system, activating a "canister full" alarm; and **characterized in that** if a difference between the first pressure and second pressure is less than a stored value in said control system memory, activating a "conduit blocked" alarm.

2. A method according to claim 1 wherein said stored value is a pressure range.

## Patentansprüche

1. Ein Verfahren zum Benachrichtigen eines Anwenders eines topischen Unterdrucktherapieapparates über einen Nicht-Strömungszustand eines Absaugfluids im Apparat, wobei der Apparat Folgendes beinhaltet: eine Vorrichtung (32) mit einem Vakuumpumpenmittel (44) und einen mit der Vorrichtung verbundenen Abfallbehälter (22); wobei der Abfallbehälter durch ein Absaugschlauchmittel (12, 24) zum Absaugen von Fluid aus der Wunde betriebsfähig mit einem Wundverband (14) verbunden ist; wobei das Absaugschlauchmittel, der Abfallbehälter und die Vorrichtung einen Fluidströmungsweg dadurch bereitstellen und das Vakuumpumpenmittel eine Fluidströmung dadurch bereitstellt; wobei die Vorrichtung ein erstes Drucksensormittel (402) zwischen einer Auslassöffnung für abgesaugtes Fluid aus dem Abfallbehälter und dem Pumpenmittel aufweist; wobei der Abfallbehälter ein damit assoziiertes zweites Drucksensormittel (422, 452) aufweist; und ein Steuerungssystem (60) zum Empfangen von Signalen von dem ersten und dem zweiten Drucksensormittel und zum Initiieren von Steuerungssignalen als Reaktion auf die Signale und Aktivieren eines passenden Anwenderalarms, Messen von Druck an dem ersten Drucksensor und an dem zweiten Drucksensor; Vergleichen des ersten und des zweiten Drucks; wenn ein Druckunterschied zwischen dem ersten und dem zweiten Druck größer ist als ein in einem Speicher des Steuerungssystems gespeicherter Wert, Aktivieren eines "Behälter voll"-Alarms; und **dadurch gekennzeichnet, dass**, wenn ein Unterschied zwischen dem ersten Druck und dem zweiten Druck geringer ist als ein im Steuerungssystemspeicher gespeicherter Wert, Aktivieren eines "Schlauch blockiert"-Alarms.

2. Verfahren gemäß Anspruch 1, wobei der gespeicherte Wert ein Druckbereich ist.

## Revendications

1. Un procédé d'alerte d'un utilisateur d'appareil de thérapie par pression négative topique d'un état de non-écoulement de fluide d'aspiration dans l'appareil, l'appareil comprenant un dispositif (32) ayant un moyen de pompe à vide (44) et un récipient à déchets (22) raccordé au dispositif ; le récipient à déchets étant raccordé fonctionnellement à un pansement pour plaie (14) par des moyens de conduit d'aspiration (12, 24) destiné à aspirer le fluide de la plaie ; les moyens de conduit d'aspiration, le récipient à déchets et le dispositif fournissant un trajet d'écoulement de fluide à travers celui-ci et le moyen de pompe à vide assurant l'écoulement de fluide à travers celui-ci ; le dispositif ayant un premier moyen de détection de pression (402) entre un orifice de sortie pour le fluide aspiré dudit récipient à déchets et ledit moyen de pompe ; ledit récipient à déchets ayant des deuxièmes moyens détecteurs de pression (422, 452) associés à lui ; et un système de commande (60) pour recevoir des signaux en provenance desdits premier et deuxième moyens détecteurs de pression et pour déclencher des signaux de commande en réponse auxdits signaux et pour activer une alerte utilisateur appropriée, pour mesurer la pression au niveau dudit premier détecteur de pression et au niveau dudit deuxième détecteur de pression ; comparer lesdites première et deuxième pressions ; si une différence de pression entre des première et deuxième pression est supérieure à une valeur stockée dans une mémoire dudit système de commande, activer une alarme « récipient plein » ; et **caractérisé en ce que**, si une différence entre la première pression et la deuxième pression est inférieure à une valeur stockée dans ladite mémoire de système de commande, activer une alarme « conduit bloqué ».

2. Un procédé selon la revendication 1 où ladite valeur stockée est une plage de pressions.
